# EUROPEAN PATENT APPLICATION

(11) **EP 4 265 324 A1**
(43) Date of publication of application: **25.10.2023**
(21) Application number: 23162735.7
(22) Date of filing: 17.03.2023
(51) Int. Cl.: B01F 29/10, B01F 33/81, B01F 33/84, B01F 33/85, B01F 35/75, B65B 3/00, B01F 101/21

(54) **APPARATUS FOR MANUFACTURING COSMETIC AND CONTROL METHOD THEREOF**

(30) Priority: 29.03.2022 KR 20220038527
(71) Applicant: Amorepacific Corporation, Seoul 04386 (KR)
(72) Inventor: Kim, Dongyoung, Yongin-si (KR); Kim, Jongwoo, Yongin-si (KR); Lee, Sanghun, Yongin-si (KR); Lee, Hyunseung, Yongin-si (KR)
(74) Representative: Dr. Schön, Neymeyr & Partner Patentanwälte mbB

(57) **Abstract**

One aspect of the present disclosure is an apparatus for manufacturing cosmetic comprising: a main body (100) providing a space for manufacturing cosmetic; a mixing vessel (161) that can store cosmetic materials and a parts supply unit (160) providing components consisting a plurality of cosmetic containers; a cosmetic material supply unit (200) that can store cosmetic materials and then discharge cosmetic materials to the mixing vessel; a transfer unit (800, 810, 820) that can transfer the mixing vessel and the components consisting the cosmetic container; and a control unit that can control the movement of the transfer unit in order to simultaneously assemble a plurality of cosmetic containers from the mixing vessel and the components.

## Description

### TECHNICAL FIELD

The present disclosure relates to an apparatus for manufacturing cosmetic and control method thereof.

### BACKGROUND

Cosmetic is used for enhancing beauty and skin health, and the need by consumers to use cosmetic that suits them is on a strong and steady increase. In order to satisfy such a need, users' apparatuses with various forms for manufacturing cosmetic are being suggested.

An apparatus for manufacturing cosmetic is generally operated in the method of measuring or diagnosing the condition of a user's skin, and accordingly calculates the blending ratio of cosmetic material appropriate for the concerned user, to mix cosmetic material based on this resulting ratio for manufacturing cosmetic. With the recent development in technology, such an apparatus for manufacturing cosmetic is being suggested as an automated apparatus that can consecutively perform a series of processes.

However, an apparatus for manufacturing cosmetic based on a related art may pose the following problems:
A problem with the prior apparatus is that it takes too much time for users to manufacture a product of their preference. Moreover, as an apparatus for manufacturing cosmetic can be provided to allow a continuous process from measuring or diagnosing a user's skin condition to manufacturing cosmetic, such a process may merely be tedious for a user to just wait for cosmetic to be manufactured in front of an apparatus for manufacturing cosmetic. Therefore, although a one or two time use out of curiosity can be expected, there is a possibility of low usage rate in terms of consistently using an apparatus for manufacturing cosmetic.

Also, delivering to an actual user the cosmetic that can be provided by an apparatus for manufacturing cosmetic may become cumbersome due to the need for performing additional tasks, such as combining of a lid by the apparatus operator to finish the assembly of a cosmetic container filled with cosmetic material, combining discharge members, and drawing out cosmetic containers from an apparatus and conveying them to the user.

Additionally, there was a time consumption issue with respect to manufacturing since an apparatus based on a related art is unable to simultaneously manufacture different types of cosmetic containers.

### [Document of Related Art]

### [Patent Document]

Patent document: Korean Patent No. 10-1299849 B1 (September 17, 2013)

### SUMMARY

The examples of the disclosure, which have been conceived to address the above-described problems, are to provide an apparatus for manufacturing cosmetic and the control method thereof that can boost the productivity of a cosmetic manufacturer or a retail store based on an automated cosmetic manufacturing process.

Additionally, another object of the present disclosure is to provide an apparatus for manufacturing cosmetic and control method thereof that allows the simultaneous assembly of different types of cosmetic containers.

Additionally, another object of the present disclosure is to provide an apparatus for manufacturing cosmetic and control method thereof that can reduce the cosmetic manufacturing time.

Additionally, another object of the present disclosure is to provide an apparatus for manufacturing cosmetic and control method thereof that allows the manufacturing of cosmetic that can be suitable for a user's skin.

According to an aspect of the present invention, there is provided an apparatus for manufacturing cosmetic comprising: a main body providing a space for manufacturing cosmetic; a parts supply unit providing a mixing vessel that can store cosmetic materials and a components consisting a plurality of cosmetic containers; a cosmetic material supply unit that can store cosmetic materials and then discharge cosmetic materials to the mixing vessel; a transfer unit that can transfer the mixing vessel and the components consisting the cosmetic container; and a control unit that can control the movement of the transfer unit in order to simultaneously assemble a plurality of cosmetic containers from the mixing vessel and the components.

Further, there is provided an apparatus for manufacturing cosmetic, wherein a multiple number of mixing vessels are provided to the parts supply unit, and the apparatus for manufacturing cosmetic further includes a mixing unit that can mix the cosmetic material stored in the mixing vessel by rotation, wherein the mixing unit includes: a first mixing unit that mixes a one mixing vessel ; and a second mixing unit that mixes another mixing vessel.

Further, there is provided an apparatus for manufacturing cosmetic, wherein the cosmetic material supply unit includes: a first cosmetic material supply unit that can discharge cosmetic material to a one mixing vessel; and a second cosmetic material supply unit that can discharge cosmetic material to another mixing vessel, wherein the respective first cosmetic material supply unit and the second cosmetic material supply unit include: a plurality of cartridges that can store cosmetic materials; a discharge nozzle that is connected to one side of respective cartridge and discharges cosmetic material to the mixing vessel; a pressure adjustment unit that can discharge the stored cosmetic material to the outside of the discharge nozzle by applying pressure on the inside of the cartridge; and a supply amount adjustment unit that adjusts the discharge amount of the cosmetic material by closing or opening the pathway in which cosmetic material moves, at the discharge nozzle.

Further, there is provided an apparatus for manufacturing cosmetic, wherein the cosmetic container includes: a first cosmetic container having a first shape; and a second cosmetic container having a second shape, wherein the parts supply unit includes: a first parts supply unit where the components consisting the first cosmetic container are arranged; and a second parts supply unit where the components consisting the second cosmetic container are arranged.

Further, there is provided an apparatus for manufacturing cosmetic, wherein the first cosmetic container is provided as a compact-type container, wherein the components consisting the compact-type container include: a base container to which the cosmetic material stored in the mixing vessel is transferred and saved; an impregnation member that is inserted in the base container to absorb cosmetic material stored in the base container; and a cover which can cover the impregnation member housed in the base container, wherein the first parts supply unit includes: a base container accommodation part on which the base container can be seated; an impregnation member accommodation part on which the impregnation member can be seated; and a first mixing vessel accommodation part on which the mixing vessel can be seated.

Further, there is provided an apparatus for manufacturing cosmetic, wherein the second cosmetic container is provided as a pump-type container, wherein the components consisting the pump-type container include: the mixing vessel storing cosmetic material; and a discharge member that covers the mixing vessel and discharges the cosmetic material stored in the mixing vessel, wherein the second parts supply unit include: a storage container seating part on which the mixing vessel can be seated; and a discharge member seating part on which the discharge member can be seated.

Further, there is provided an apparatus for manufacturing cosmetic, wherein the transfer unit includes: a first transfer unit that can assemble the first cosmetic container from the components; and a second transfer unit that can assemble the second cosmetic container from the mixing vessel and the components.

Further, there is provided an apparatus for manufacturing cosmetic includes an assembly unit providing a space in which a plurality of cosmetic containers are assembled from the mixing vessel and the components, wherein the assembly unit includes: a first assembly unit that provides a space where a first cosmetic container having a first shape is assembled; a second assembly unit that provides a space where a second cosmetic container having a second shape is assembled; a base container transfer unit that can transfer a base container which is a part of component comprising the first cosmetic container; a hygroscopic mechanism that can apply pressure on an impregnation member stored in the base container.

Further, there is provided an apparatus for manufacturing cosmetic includes, a mixing vessel transfer unit that is placed on the lower side of the cosmetic material supply unit and can transfer the mixing vessel.

Further, there is provided an apparatus for manufacturing cosmetic includes a vibrating device that can discharge the cosmetic material stored in the mixing vessel to a part of the components, after gripping the mixing vessel disposed at the mixing vessel transfer unit.

Further, there is provided an apparatus for manufacturing cosmetic, wherein the cosmetic material supply unit includes a plurality of cartridges that can store the cosmetic materials, and between the plurality of cartridges, a space for introducing a transfer unit that allows the entrance of the transfer unit gripping the mixing vessel is formed.

According to another aspect of the present invention, there is provided a control method of an apparatus for manufacturing cosmetic including, (S11) a step in which the components comprising a mixing vessel and the first cosmetic container are provided to the first parts supply unit; (S12) a step in which after the mixing vessel is gripped by the first transfer unit, the mixing vessel is transferred to a mixing vessel transfer unit placed at the bottom of the first cosmetic material supply unit; (S13) a step in which the mixing vessel transfer unit is transferred along the first cosmetic material supply unit, to discharge the multiple number of cosmetic materials stored in the first cosmetic material supply unit to the mixing vessel; (S14) a step in which after the mixing vessel arranged at the mixing vessel transfer unit is gripped by the first transfer unit, the mixing vessel is transferred to the first mixing unit, and then the first mixing unit is rotated to mix the cosmetic material stored in the mixing vessel; (S15) a step in which after the mixing vessel arranged at the first mixing unit by the first transfer unit is transferred to a mixing vessel transfer unit, the cosmetic material stored in the mixing vessel is discharged as a part of the components consisting the first cosmetic container.

Further, there is provided a control method of an apparatus for manufacturing cosmetic including, (S21) a step in which the mixing vessel and the components consisting the second cosmetic container are provided to the second parts supply unit; (S22) a step in which after the mixing unit is gripped by the second transfer unit 820, the mixing unit 161 is transferred to the mixing unit transfer unit placed at the bottom of the second cosmetic material supply unit; (S23) a step in which the mixing vessel transfer unit is transferred along the second cosmetic material supply unit to discharge the multiple number of cosmetic material stored in the second cosmetic material supply unit to the mixing vessel; (S24) a step in which after the mixing vessel placed at the mixing vessel transfer unit is gripped by the second transfer unit, the mixing vessel is then transferred to the second mixing unit, to rotate the second mixing unit for mixing the cosmetic material stored in the mixing vessel 161; (S25) a step in which after the mixing vessel disposed at the second mixing unit by the second transfer unit is transferred to an assembly unit, the second cosmetic container is formed by assembling a multiple number of the components.

Further, there is provided a control method of an apparatus for manufacturing cosmetic, wherein the S11 to S 16, and the S21 to 25 are simultaneously implemented.

An apparatus for manufacturing cosmetic and control thereof, according to these embodiments, holds an advantage in that the manufacturing process of cosmetic is automated to enhance the productivity of a cosmetic manufacturer or a retail store.

Moreover, these embodiments are advantageous in that the manufacturing time can be reduced through a simultaneous assembly of different types of cosmetic containers.

Furthermore, these embodiments offer an advantage in that cosmetic suitable for the condition of a user's skin can be manufactured.

### BRIEF DESCRIPTION OF DRAWINGS

Fig.1 is a conceptual drawing of an apparatus for manufacturing cosmetic 1 in accordance with the embodiment of the present invention.
Fig. 2 is a perspective view showing the appearance of an apparatus for manufacturing cosmetic 10 according to the embodiment of the present invention.
Fig. 3 is a perspective view referring to the internal structure of an apparatus for manufacturing cosmetic 10 in Fig.2.
Fig. 4 is a top perspective view, where the inside of an apparatus for manufacturing cosmetic in Fig.3 is viewed from the upper side.
Fig. 5 is a drawing that illustrates in detail a cosmetic material supply unit 200, a mixing vessel transfer unit 250, and a vibrating device 300 in an apparatus for manufacturing cosmetic in Fig. 3.
Fig.6 is a perspective view of a mixing vessel transfer unit 250 in an apparatus for manufacturing cosmetic in Fig.3
Fig. 7 is a perspective view of an assembly unit 50 of an apparatus for manufacturing cosmetic in Fig. 3.
Fig. 8 is a perspective view showing the first parts supply unit 162 in an apparatus for manufacturing cosmetic 10 in Fig.3 and components that can be placed therein.
Fig. 9 is a perspective view representing the second parts supply unit 164 in an apparatus for manufacturing cosmetic in Fig.3 and components that can be placed therein.
Fig. 10 is a perspective view referring to a transfer unit 800 in an apparatus for manufacturing cosmetic in Fig. 3.
Fig. 11 is a perspective view illustrating a vibrating device 300 in an apparatus for manufacturing cosmetic 10 in Fig. 3.
Fig. 12 is an exploded perspective view of a vibrating device 300 in Fig. 11.
Fig. 13 is a flowchart depicting the control method of an apparatus for manufacturing cosmetic in Fig. 2.

### DETAILED DESCRIPTION OF EXEMPLARY EMBODIMENTS

The details on the specific embodiments of the present invention are hereunder thoroughly explained with reference to drawings. When explaining the present invention, if it is deemed that a detailed explanation on the relevant publicly known construction or function can obscure the essence of the present invention, such detailed explanation will be omitted.

In the explanation hereunder, a "cosmetic"can be understood as a product that directly or indirectly works on a user's skin to provide a cosmetic effect to the skin, protect the skin, enhance or change skin condition, or perform any or more of these functions, and "cosmetic material"
can be understood as a material comprising such cosmetic. Also, "cosmetic container" can be understood as a container housing cosmetic.

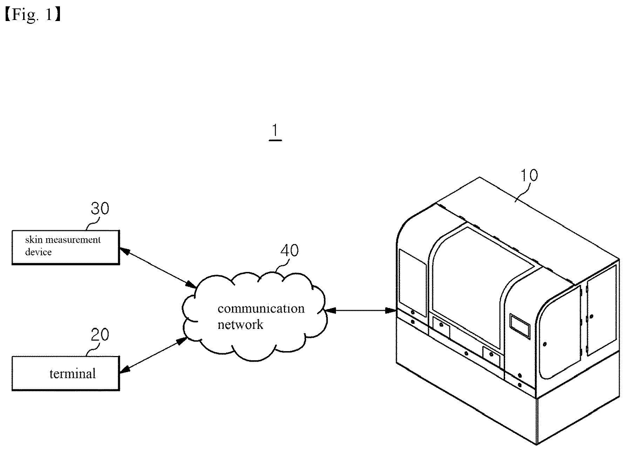

Fig. 1 is a drawing conceptually representing a system for manufacturing cosmetic 1 with accordance to the first embodiment of the present invention.

With reference to Fig. 1, a system for manufacturing cosmetic 1 according to an embodiment of the present disclosure may include, a skin measurement device 30 that can measure a user's skin condition; a terminal that can receive information on user context 20; and an apparatus for manufacturing cosmetic that can manufacture cosmetic 10.

Here, an apparatus for manufacturing cosmetic 10, a terminal 20, and a skin measurement device 30, can be provided in a way where communication is possible among them through a communication network 40.

A system for manufacturing cosmetic and an apparatus for manufacturing cosmetic 10 in this present embodiment, can manufacture customized cosmetic.

Here, customized cosmetic can be understood as cosmetic that has been manufactured based on a composition ratio determined in line with a user's skin condition, or the analysis or diagnosis of such skin condition.

A skin measurement device 30, an instrument for measuring a user's skin condition, can include all skin measuring devices such as a camera, moisture sensor, and oil sensor.

When a skin measurement device 30 is provided as a camera, a user's skin condition can be assessed based on the filmed skin image.

The condition of a user's skin measured by a skin measurement device 30 can be delivered to an apparatus for manufacturing cosmetic 10 through a terminal 20, or can be directly delivered to an apparatus for manufacturing cosmetic 10.

A terminal 20 can receive information on user context, and the information received can be transmitted to an apparatus for manufacturing cosmetic 10.

A terminal 20 can, through a skin measurement device 30, transmit the measured skin condition of the user to an apparatus for manufacturing cosmetic 10, or analyze such measurement to allow transmission of diagnosed skin condition data to an apparatus for manufacturing cosmetic 10.

This present embodiment uses an example in which a terminal 20 and a skin measurement device 30 are provided as separate configurations, but a terminal 20 and a skin measurement device 30 can also be provided as a whole configuration.

An apparatus for manufacturing cosmetic 10 can determine the composition ratio of cosmetic material needed for manufacturing cosmetic based on data transmitted from a terminal 20 or a skin measurement device 30.

The control unit of an apparatus for manufacturing cosmetic 10 can, based on a user's skin condition or user context, determine the appropriate composition ratio for each user.

Here, user context means data in relevance to a user's life such as a user's biometric information, spatial information of a user's surroundings, preset time range and preset spatial range in relation to a user's schedule, shopping history of a user, purchase history of a user, and a user's online activity record, etc., that may be regarded as information which can be transmitted from any equipment or a third party.

An apparatus for manufacturing cosmetic 10 can receive a user's information to manufacture cosmetic from a terminal 20, from a skin measurement device 30, or from both, and can manufacture cosmetic based on transmitted information.

An apparatus for manufacturing cosmetic 10 can simultaneously assemble cosmetic containers of different shapes(e.g. the first cosmetic container and the second cosmetic container).

Although this present embodiment provides an example of an apparatus for manufacturing cosmetic 10 assembling two types of cosmetic containers(the first cosmetic container, the second cosmetic container), the assembly of different cosmetic container types can be included, which will be elaborated further below.

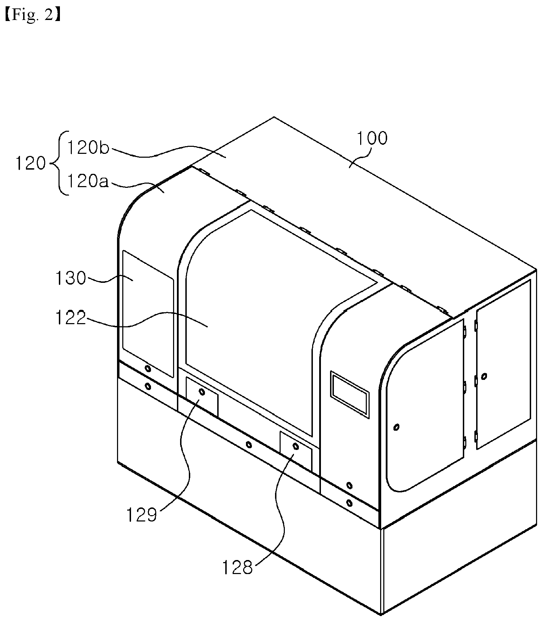

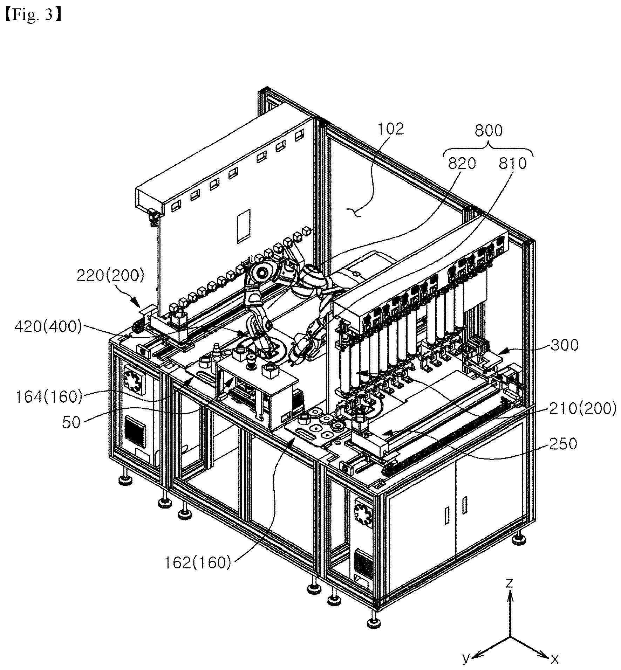

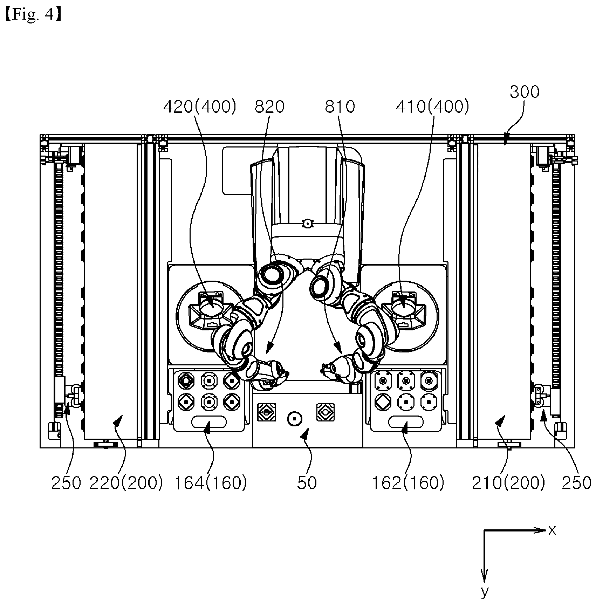

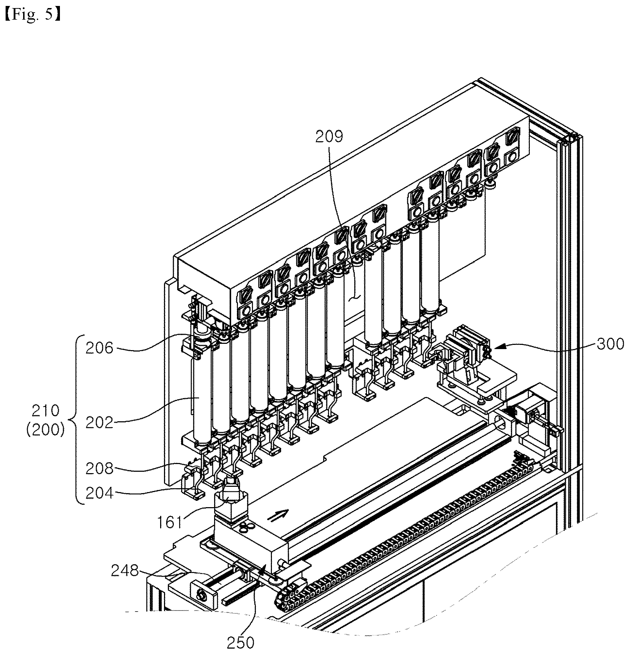

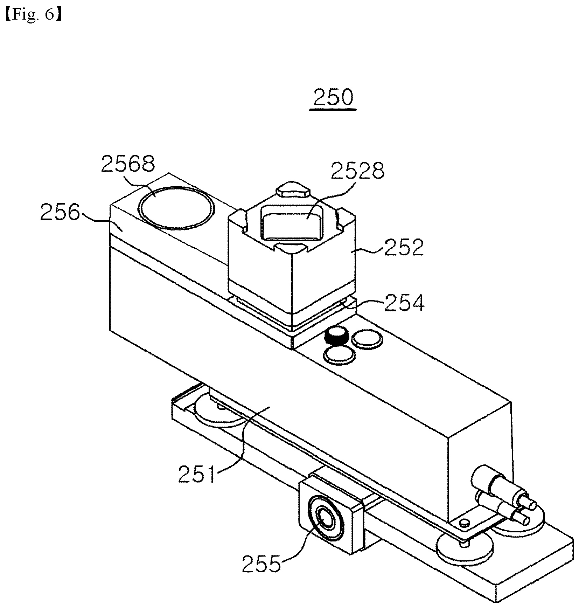

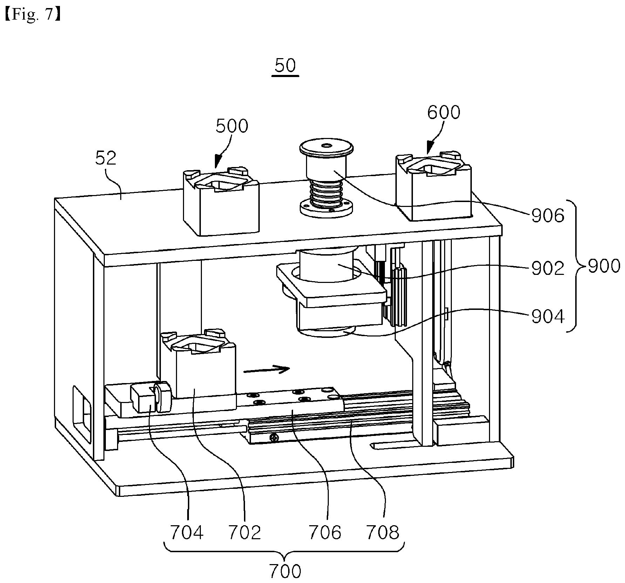

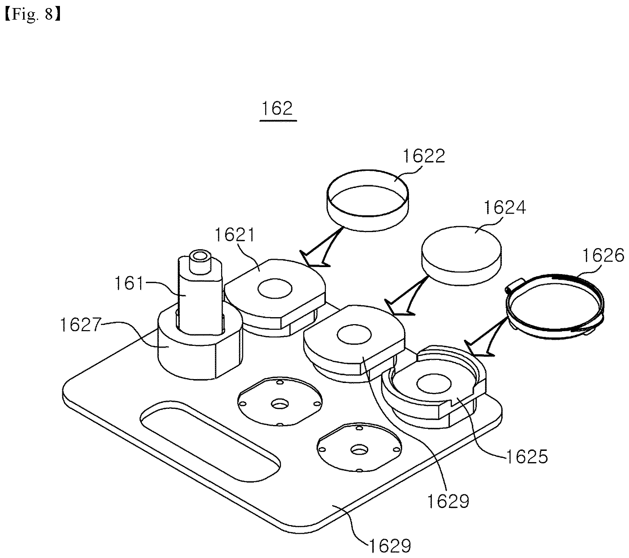

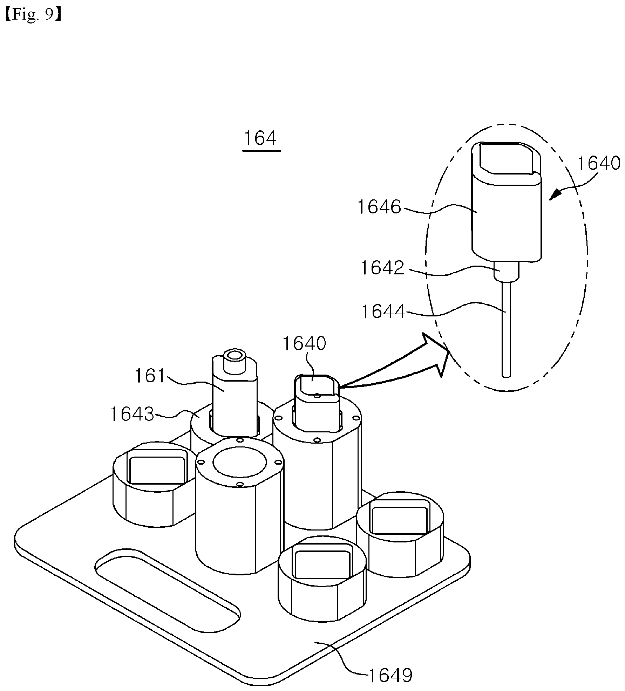

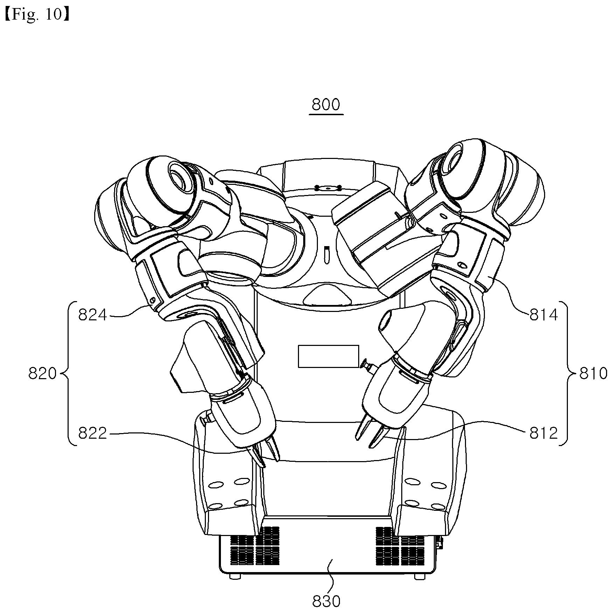

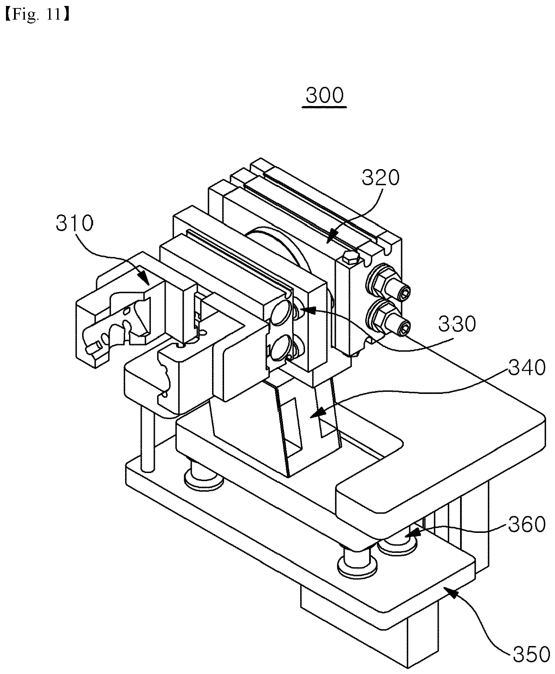

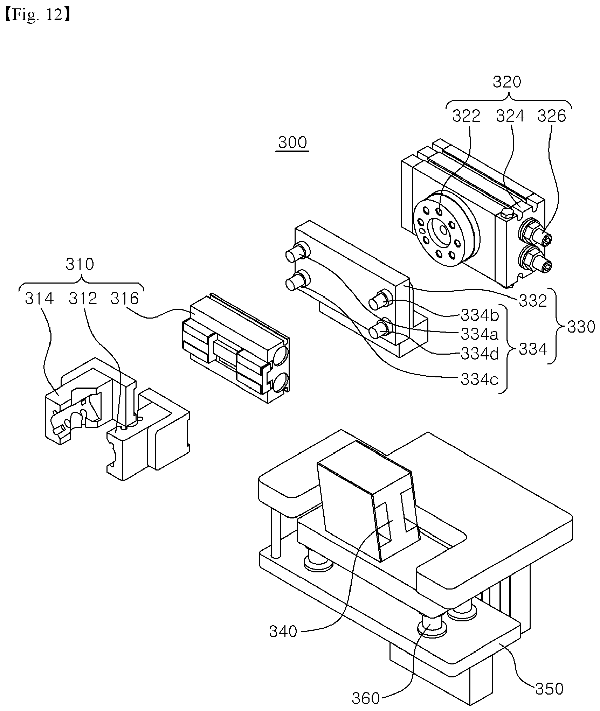

Fig.2 is a perspective view describing the appearance of an apparatus for manufacturing cosmetic 10 in accordance with the embodiment of the present invention; Fig.3 is a perspective view describing the internal structure of an apparatus for manufacturing cosmetic in Fig.2; Fig.4 is a top perspective view that views the inside of an apparatus for manufacturing cosmetic in Fig.3 from the upper side; Fig.5 is a drawing that describes in further detail a cosmetic material supply unit 200, a mixing vessel transfer unit 250, and a vibrating device 300 of an apparatus for manufacturing cosmetic 10 in Fig.3; Fig. 6 is a perspective view of a mixing vessel transfer unit 250 of an apparatus for manufacturing cosmetic in Fig.3; Fig. 7 is a perspective view of an assembly unit 50 of an apparatus for manufacturing cosmetic 10 in Fig.3; Fig. 8 is a perspective view representing the first parts supply unit 162 and components that can be placed therein in Fig.3; Fig.9 is a perspective view representing the second parts supply unit 164 and components that can be placed therein in Fig. 3; Fig. 10 is a perspective view representing a transfer unit 800 of an apparatus for manufacturing cosmetic in Fig.3; Fig. 11 is a perspective view representing a vibrating device 300 of an apparatus for manufacturing cosmetic in Fig.3; Fig. 12 is an exploded perspective view of a vibrating device 300 in Fig. 11.

Referring to Fig. 2 to Fig. 12, an apparatus for manufacturing cosmetic 10 can be provided to simultaneously assemble a multiple number of cosmetic containers with different forms(e.g. the first cosmetic container and the second cosmetic container).

An apparatus for manufacturing cosmetic 10 can include: a main body 100 providing a space for manufacturing cosmetic 102; a parts supply unit 160 providing a mixing vessel 161 that can house cosmetic material and components for the composition of a multiple number of cosmetic containers; a cosmetic material supply unit 200 that can, after storing a multiple number of cosmetic material, discharge cosmetic material to the mixing vessel 161; a transfer unit 800 that can transfer a mixing vessel 161 and the components comprising a cosmetic container; and a rotary unit 400 provided as rotatable in order to mix the cosmetic material housed in a mixing vessel 161; an assembly unit 50 providing a space for assembling a cosmetic container from a mixing vessel 161 and components; a transfer unit 800 that allows a transfer of a mixing vessel 161 and component from any one of the parts supply unit 160, cosmetic material supply unit 200, mixing unit 400, assembly unit 50 to any one of these four units; and a control unit that can simultaneously assemble a multiple number of cosmetic containers storing cosmetic by controlling at least one among a cosmetic material supply unit 200, mixing unit 400, and transfer unit 800.

Moreover, an apparatus for manufacturing cosmetic 10 further includes, a mixing vessel transfer unit 250 that can transfer a mixing vessel 16 and is arranged on the lower side of a cosmetic material supply unit 200; and a vibrating device 300 that can discharge the cosmetic material stored in a mixing vessel 161 to some part of the components after gripping the mixing vessel 161 which is arranged on the mixing vessel transfer unit 250.

The main body 100, forming the appearance of an apparatus for manufacturing cosmetic 10, can provide a space for manufacturing cosmetic 102 where components (e.g. parts supply unit 160, cosmetic material storage unit 140, cosmetic material supply unit 200, mixing unit 400, transfer unit 800, assembly unit 50) for manufacturing cosmetic can be installed.

In order to provide a space for manufacturing cosmetic 102, a main body 100 can take on any three-dimensional shape, and this present embodiment illustrates an example where an overall rectangular parallelepiped shape is formed.

The abovementioned cosmetic material storage unit 140, parts supply unit 160, cosmetic material supply unit 200, mixing unit 400, transfer unit 800 and assembly unit 50 can be placed in a space for manufacturing cosmetic 102, and these components can be placed on the bottom surface of a space for manufacturing cosmetic 102.

According to the embodiment, the components described above may be installed on any wall surface forming a space for manufacturing cosmetic 102.

When looking at an apparatus for manufacturing cosmetic 10 from the upper side(see Fig.4), a cosmetic supply unit 200 can be placed at both sides of a main body 100; a parts supply unit 160 can be placed at the left-center of the front side and the right-center of the front side; an assembly unit 50 can be placed at the front-center; a mixing unit 400 can be respectively placed at the rear side of a parts supply unit, and a transfer unit 800 can be placed between a multiple number of mixing unit 400, and a vibrating device can be placed at the rear side of a cosmetic supply unit 200.

Here, the front side, based on Fig. 4, can be understood as the +y direction and the right side as the +x direction.

A main body 100 can include a housing 120 in order to protect a space for manufacturing cosmetic 102 from the outside.

Specifically, a housing 120 can include a front housing 120a that surrounds the front of a space for manufacturing cosmetic 102; and a back housing 120b that surrounds the back of a space for manufacturing cosmetic 102.

A housing 120 can include a transparent window 122 providing transparency, to allow a space for manufacturing cosmetic 102 to be seen.

The front-central area of a housing 120 can be formed with a transparent window 122, and the user can, through a transparent window 122, see a cosmetic container being assembled.

In other words, a transparent window 122 allows a user to see the process of mixing cosmetic material and assembling cosmetic containers conducted in a space for manufacturing cosmetic 102, resulting in the improvement of user trust on cosmetic manufactured through an apparatus for manufacturing cosmetic 10.

A user interface unit 130 can be provided to one side of a housing 120.

A user interface unit 130 can provide to a user the state information of an apparatus for manufacturing cosmetic 10, process of manufacturing cosmetic and relevant information thereof, and can be provided so as to receive preset information from a user. For instance, a user interface unit 130 can be a touch screen, and can include a microphone and speaker for input and output of audio. The location where a user interface unit 130 is provided on a housing 120 can be configured to prevent an overlap with a transparent window 122, and the present embodiment illustrates this with an example of being provided to the front-left area of a main body 100.

The user can, through a user interface unit 130, can implement actions such as analyzing one's own skin condition, checking diagnosed result, checking the composition ratio of cosmetic to be provided to oneself, adjusting the composition ratio of cosmetic to be manufactured, or selecting the cosmetic having a specific composition ratio.

In addition, a housing 120 can include the first door 128 for supplying the components consisting a cosmetic container (e.g. the first cosmetic container) to the first parts supply unit 162; and the second door 129 to supply the components consisting the cosmetic container(e.g. the second cosmetic container) to the second parts supply unit 164.

The first door 128 and the second door 129 can be provided in a rotary structure having elastic restoring force or a slide structure.

A cosmetic material supply unit 200 can, after storing a multiple number of cosmetic materials, discharge the materials to a mixing vessel 161.

A cosmetic material supply unit 200 can be provided in multiple numbers. In this present embodiment, a cosmetic material supply unit 200 is explained by taking an example of being provided to both sides of a transfer unit 800. Specifically, one cosmetic material supply unit 200 can supply the cosmetic material housed in the first cosmetic container, and the other cosmetic material supply unit 200 can supply the cosmetic material housed in the second cosmetic container.

Respective cosmetic material 200 can include, a multiple number of cartridges that can house a multiple number of cosmetic materials 202; a discharge nozzle 204 that is connected to one side of respective cartridges and discharges cosmetic material to a mixing vessel 161; a pressure adjusting unit 206 that can discharge the cosmetic material stored in a cartridge 202 to the outside of a discharge nozzle 204 by applying pressure on the inside of a cartridge 202; and a supply amount adjusting unit 208 that adjusts the discharge amount of the cosmetic material by closing or opening the path in which the cosmetic material moves within a discharge nozzle 204(See. Fig. 5).

Cosmetic material used as the material of cosmetic can be stored in a respective cartridge 202, and different types of cosmetic material can be stored in different cartridges 202.

A pressure adjusting unit 206, as an air dispenser, can apply pressure to the inside of a cartridge 202 by being attached to the end of a respective cartridge 202.

By pressing both sides of a discharge nozzle 204, a supply amount adjusting unit 208 can close the path in which cosmetic material moves, and by releasing the press on a discharge nozzle 204, the path in which cosmetic material moves can be opened.

The cosmetic material supply unit 200, based on the composition ratio generated by a control unit, can discharge the cosmetic material stored in a cartridge 202 to a mixing vessel 161 arranged at a mixing vessel transfer unit 250. Here, the composition ratio is preset and stored in the memory(not shown), or is delivered from a terminal 20, or can be calculated by a control unit based on the data received from a terminal 20.

The speed of discharging cosmetic material from a cosmetic material supply unit 200 may vary based on the control of a control unit.

For example, a control unit can control a pressure adjusting unit 206 that can adjust the pressure applied to a cartridge 202 and a supply amount adjusting unit 208 that can adjust the size of the path in which the cosmetic material of a discharge nozzle 204 moves.

Among a multiple number of cartridges 202, a space for introducing a transfer unit 209 that can allow the entrance of a transfer unit 800 gripping a mixing vessel 161 can be formed.

A multiple number of cartridges 202 are to be spaced regularly, while a space for introducing a transfer unit 209 into which a transfer unit 800 can be introduced can be formed.

After introducing a transfer unit 800 gripping a mixing vessel 161 through a space for introducing a transfer unit, a mixing vessel 161 is seated on a mixing vessel transfer unit 250 and the mixing vessel transfer unit 250 is unidirectionally moved from the bottom of a cosmetic material supply unit 200, and cosmetic material can be discharged from the cosmetic material supply unit 200 to a mixing vessel 161 in the mixing vessel transfer unit 250.

A door(not shown in drawing) can be provided to a space for introducing a transfer unit 209, and the door can be moved to either open or close a space for introducing a transfer unit 209.

A mixing vessel transfer unit 250, which can be movable, can be provided to the lower side of a cosmetic material supply unit 200.

A mixing vessel transfer unit 250, which can be movable, can be provided along the direction(e.g. y-axis direction) to which a cosmetic material supply unit 200 is arranged.

One side of a mixing vessel transfer unit 250 can be connected with a transfer guide unit 248 that provides the power to move a mixing vessel transfer unit 250, and a transfer guide unit 248 can be extended to the direction to which a cosmetic material supply unit 200 is arranged.

A mixing vessel transfer unit 250 can move along a transfer guide unit 248, and when a transfer guide unit 248 rotates to one side, a mixing vessel transfer unit 250 can move to one side(e.g. y-axis direction), and when a transfer guide unit 248 rotates in reverse direction, a mixing vessel transfer unit 250 can move to another direction(e.g. -y-axis direction).

Specifically, a mixing vessel transfer unit 250 can include a mixing vessel seating part 252 on which a mixing vessel 161 transferred by a transfer unit 800 can be seated; and a weight detecting unit 254 that can detect cosmetic material housed in a mixing vessel 161 seated on a mixing vessel seating part 252 (See Fig. 6).

Furthermore, one side of a main body of a transfer unit 251 can include a coupling unit 255 coupled with a transfer guide unit 248.

A coupling unit 255 can change the rotational motion of a transfer guide unit 248 to a linear motion of a main body of a transfer unit 251.

Also, a mixing vessel transfer unit 250 can additionally include a component seating part 256 on which is seated the component consisting the first cosmetic container(e.g. compact-type container).

When a component is seated on a component seating part 256, a mixing vessel 161 seated on a mixing vessel seating part 252 by the following vibrating device 300 is turned upside down to transfer cosmetic material stored in a mixing vessel 161 to the component seated on a component seating part 256. This process will be explained in detail in the following sections.

Moreover, a mixing vessel seating part 252 and a component seating part 256 can be placed at the upper part of a main body of a transfer unit 251, while the first home 2528 into which a mixing vessel 161 can be inserted can be formed on a mixing vessel seating part 252, and the second home 2568 into which a component having a preset shape(e.g. cylinder shape)can be inserted can be formed on a component seating part 256.

A mixing vessel transfer unit 250 can, after waiting for a preset amount of time at the lower part of a discharge nozzle 204 in a cosmetic material supply unit 200, be moved to the lower part of the next discharge nozzle 204. While a mixing vessel transfer unit 250 waits for a preset amount of time, cosmetic material can be discharged from a discharge nozzle 204 to a mixing vessel 161.

At this point a weight detection unit 254 detects the change in the weight of a mixing vessel 161 disposed at a mixing vessel transfer unit 250, to detect the weight of cosmetic material discharged from a cosmetic material supply unit 200 to a mixing vessel 161. At this moment, a control unit can control the supply speed of cosmetic material through a cosmetic material supply unit 200 based on the respective weight of cosmetic material measured by a weight detection unit 254.

A parts supply unit 160 can include the first parts supply unit 162 on which the component consisting the first cosmetic container is placed; and the second parts supply unit 164 on which the component consisting the second cosmetic container is placed(See Fig.8 and Fig. 9).

Here, a cosmetic container can include the first cosmetic container having the first shape and the second cosmetic container having the second shape.

In this present embodiment, it is explained by way of example that the first cosmetic container having the first shape is a compact-type container and that the second cosmetic container having the second shape is a pump-type container.

A component and mixing vessel 161 consisting a compact-type container as the first cosmetic container can be placed at the first parts supply unit 162(See. Fig. 8).

The component comprising a compact-type container, which is the first cosmetic container, can include a base container 1622 into which cosmetic material housed in a mixing vessel 161 is moved and stored; an impregnation member 1624 that is inserted in a base container 1622 to absorb cosmetic material stored in a base container 1622; and a cover 1626 that can cover an impregnation member 1624 housed in a base container 1622.

The first parts supply unit 162 can provide a space at which such a base container 1622, impregnation chamber 1624, cover 1626, and mixing vessel 161 can be placed. Specifically, the first parts supply unit 162 provides a space on which a component can be seated and can include the first tray that can be slid 1629; a base container accommodation part 1621 that is placed on the first tray 1629 and on which a base container 1622 can be seated; an impregnation member accommodation part 1624 that is disposed at the top of the first tray 1629 and be seated with an impregnation member 1624; a cover accommodation part 1625 that is disposed at the top of the first tray 1629 and on which a cover 1626 can be seated; and the first mixing vessel accommodation part 1627 that is disposed at the top of the first tray 1629 and on which a mixing vessel 161 can be seated.

A component and a mixing vessel 161 consisting a pump-type container as the second cosmetic container can be arranged at the second parts supply unit 164.

A component consisting a pump-type container, which is the second cosmetic container, can include a storage container that stores cosmetic material; and a discharge member 1640 that can cover the storage container, and discharge cosmetic material stored in a storage container. Here, a storage container can be provided as a mixing vessel 161, or as a separate container.

A mixing vessel 161 is not only used to mix cosmetic material, but can also be used as a partial component of the second cosmetic container.

A discharge member 1640 can include a pumping part 1642 that provides pressure to transfer cosmetic material by generating a pressure difference; a straw 1644 that is connected to a pumping part 1642 and can suck in cosmetic material; and a cap 1646 that can close the inlet of a storage container.

The second parts supply unit 164, can include the second tray 1649 that can be moved by sliding which providing a space on which a component can be seated; a storage container accommodation part 1643 that is placed at the second tray 1649 and on which a storage container can be seated; and a discharge member accommodation part 1645 that is placed at the second tray 1649 and on which a discharge member 1640 can be seated.

In case a storage container is a mixing vessel 161, a mixing vessel 161 can be seated on a storage container accommodation part 1643.

A mixing unit 400 can be provided as rotatable in order to mix the cosmetic material stored in a mixing container 161.

A mixing unit 400 can be provided in multiple numbers. This present embodiment explains by taking the example of a mixing unit 400 being provided to both sides of a transfer unit 800.

Specifically, a mixing unit 400 can include the first mixing unit 410 that mixes a mixing vessel 161; and the second mixing unit 420 that mixes another mixing vessel 161.

A mixing unit 400 is explained through the example that it is a device mixing cosmetic material by simultaneously revolving and rotating a mixing vessel 161 at high speed.

Specifically, a respective first mixing unit 410 and second mixing unit 420 can include, the first rotary member(not shown in drawing) that is rotatable and into which a mixing vessel 161 can be inserted; and the second rotary member(not shown in drawing) that can rotate the first rotary member. Here, the first rotary member can be spaced apart for some distance at the center of the second rotary member, and the first rotary member can stop at a location preset by a control unit.

In this case, as a transfer unit 800 can insert a mixing vessel 161 into the first rotary member situated at a present location, it can enable an easier control of a transfer unit 800.

A mixing unit 400, after housing a mixing vessel 161, can be operated up to a preset amount of time in accordance with the control of a control unit(not shown in drawing), and when the mixing process in a mixing unit 400 is completed, a mixing vessel 161 can be transferred to the subsequent process location by a transfer unit 800.

An assembly unit 50 can provide a space where a multiple number of cosmetic containers can be assembled from a mixing vessel 161 and component(see Fig. 7).

Specifically, an assembly unit 50 can include, the first assembly unit 500 that provides a space in which the first cosmetic container having the first shape can be assembled; the second assembly unit 600 that provides a space in which the second cosmetic container having the second shape can be assembled; a base container transfer unit 700 that can transfer a base container 1622 consisting the first cosmetic container; and a hygroscopic device 900 that can apply pressure on an impregnation chamber 1624 stored in a base container 1622.

The first assembly part 500 and the second assembly part 600 are placed on the upper part of a table 52, and a base container transfer unit 700 and hygroscopic device 900 can be placed at the lower part of a table 52.

The first assembly part 500 can provide a space on which a base container 1622 storing cosmetic material can be seated.

The first cosmetic container can be completed when a base container 1622 storing cosmetic material is seated on the first assembly unit 500 and then a base container 1622 and cover 1626 are assembled.

The second assembly unit 600 can provide a space on which a mixing vessel 161 storing cosmetic material can be seated.

The second cosmetic container can be completed when a mixing vessel 161 storing cosmetic material is seated on the second assembly unit 600, and then a mixing vessel 161 and discharge member 1640 are assembled.

A base container transfer unit 700 and hygroscopic mechanism 900 can be comprehended as a mechanism that mixes an impregnation member 1624 and cosmetic material stored in a base container 1622.

Specifically, by pressing through a pressing portion 902 an impregnation member 1624 stored in a base container 1622, cosmetic material and impregnation member 1624 stored in a base container 1622 can be mixed.

A base container transfer unit 700 can include, a base container seating part 702 on which a base container 1622 storing cosmetic material can be seated; a protrusion member 704 that can be gripped and transferred by a transfer unit 800; the first guide member 706 that is extended to one side(x-axis direction); the second guide member 708 arranged at the lower side of a table 52 for guiding the first guide unit 706.

Here, the second guide member 708 is fixated, and the first guide member 706 can move reciprocally along the second guide member 708.

A hygroscopic mechanism 900 can include a pressing portion 902 having a size enough to be accommodated in the inside of a base container 1622; a support portion 904 having a size enough to support the outer side of a base container 1622, and a pressing portion 902 and support portion 904 are relatively and vertically movable.

Specifically, when a base container 1622 is placed at the lower part of a hygroscopic mechanism 900, a support portion 904 descends to fixate the outer side of a base container 1622, and a pressing portion 902 descends to apply pressure on an impregnation chamber 1624 housed in the inside of a base container 1622, to mix an impregnation member 1624 with cosmetic material.

Furthermore, a hygroscopic mechanism 900 can selectively include a pushing portion 906 that can be pushed by a transfer unit 800.

When the first conveying unit 810 or the second conveying unit 820 of a conveying unit 800 pushes a pushing portion 906, the impregnation member 1624 pressed as the pressing portion 902 descends.

A vibrating device 300 can, after gripping a mixing vessel 161 disposed at a mixing vessel transfer unit 250, discharge cosmetic stored in a mixing vessel 161 to a base container 1622 which is a part of the components.

A vibrating device 300 can include, a vibrating gripper 310 that can grip a mixing vessel 161 storing cosmetic material; a rotary unit 320 that can rotate the vibrating gripper 310 to tilt a mixing vessel 161 gripped by a vibrating gripper 310; and a vibration generator unit 330 that can apply vibration to a vibrating gripper 310, in order to discharge cosmetic material stored in a mixing vessel 161 to the outside of a mixing vessel 161

A vibrating gripper can include, the first vibrating gripper 312 that can come into contact with a part of a mixing vessel 161; and the second vibrating gripper 314 that can come into contact with another part of a mixing vessel 161. Also, the first vibrating gripper 312 and the second vibrating gripper 314 can move close or far from each other.

Moreover, a vibrating gripper 310 can further include a gripper adjusting member 316 that can move the first vibrating gripper 312 and the second vibrating gripper 314.

A rotary unit 320 can include a rotary member 322 that is connected with a vibration generating unit 330 and is provided as 360 degrees rotatable.

Furthermore, a rotary unit 320 can include a main body 324 of a rotary unit that is fixed onto a fixing unit 340; and a pressure adjustment inlet 326 that is connected to one side of a main body 324 of a rotary unit and introduces air pressure that provides the driving force to rotate a rotary member 322.

A rotary member 322 connects a main body of a rotary unit 324 and a main body of a vibration generation unit 332, and a vibrating gripper 310 can be rotated by rotating a rotary member 322.

Although a rotary member 322 was illustrated by the example of it being formed as a cylinder shape, this does not mean that the shape of a rotary member 322 is limited.

A vibration generation member 330, can include a main body of a vibration generation unit 332 that is connected to a rotary unit 320; and a multiple number of vibrating members 334 that connects a main body 332 of a vibration generation unit and the vibrating gripper 310 and can respectively move in different directions.

Respective vibrating members 334 vibrate in the frequency of 40Hz or 400Hz, or preferably, in the frequency of 60Hz.

Here, a vibrating member 334 can be provided as a feeder coil, and the frequency can be controlled variably by a control unit(not shown in drawing).

Furthermore, a vibrating mechanism 300 can include a height adjusting unit 360 provided to change the length for adjusting the height of a rotary unit 320. Even though a mixing vessel 161 of various sizes become available with the provision of a height adjusting unit 360, a vibrating gripper 310 can easily grip a mixing vessel 161. A height adjusting unit 360 can be provided between a lower base 350 and a fixing unit 340.

Even in the case where high viscosity cosmetic material is stored in a mixing vessel 161 by a vibrating device 300, cosmetic can be easily discharged by applying vibration to a mixing vessel 161.

A transfer unit 800 can include, the first transfer unit 810 that can assemble the first cosmetic container; and the second transfer unit 820 that can assemble the second cosmetic container from a mixing vessel 161 and component.

The first transfer unit 810 can be used to assemble the first cosmetic container, and the second transfer unit 820 can be used to assemble the second cosmetic container. Moreover, as the first transfer unit 810 and the second transfer unit 820 can move independently, the first cosmetic container and the second cosmetic container can be formed simultaneously or with a time gap.

The first transfer unit 810 and the second transfer unit 820 are connected to a robotic arm control unit 830, and can have a shape of being extended from a robotic arm control unit 830. A robotic arm control unit 830, in accordance with the control by a control unit(not shown in drawing), can allow the implementation of preset functions by controlling the first transfer unit 810 and the second transfer unit 820.

The first gripper 812 is provided to the first transfer unit 810, and the second gripper 822 is provided to the second transfer unit 820. The first gripper 812 and the second gripper 822 can respectively have a structural form appropriate for the functions to be conducted by the first transfer unit 810 and the second transfer unit 820.

The first transfer unit 810 can include the first multi-joint arm 814 that moves in a multiple number of directions; and the first gripper 812 provided to the end of the first multi-joint arm 814. The second transfer unit 820 can include the second multi-joint arm 824 that moves in multiple directions; and the second gripper 822 provided to the end of the second multi-joint arm 824.

A control unit(not shown in drawing) can basically control all the components described above.

A control unit(not shown in drawing), deciding the composition ratio of cosmetic to be manufactured, can control a transfer unit 800, cosmetic supply unit 200, mixing vessel transfer unit 250, vibrating device 300, mixing unit 400, base container transfer unit 700, and a hygroscopic mechanism 900.

For example, a control unit(not shown in drawing) controls the movement of the first transfer unit 810 and the second transfer unit 820, adjusts the amount and speed of cosmetic material being discharged from the first cosmetic material supply unit 210 and the second cosmetic material supply unit 220, controls the rotation angle and frequency of a vibrating gripper 310 of a vibrating device 300, controls the rotation speed of and stopping location in the first mixing unit 410 and the second mixing unit 420, and controls the pressure of a hygroscopic mechanism pressing a base container 1622.

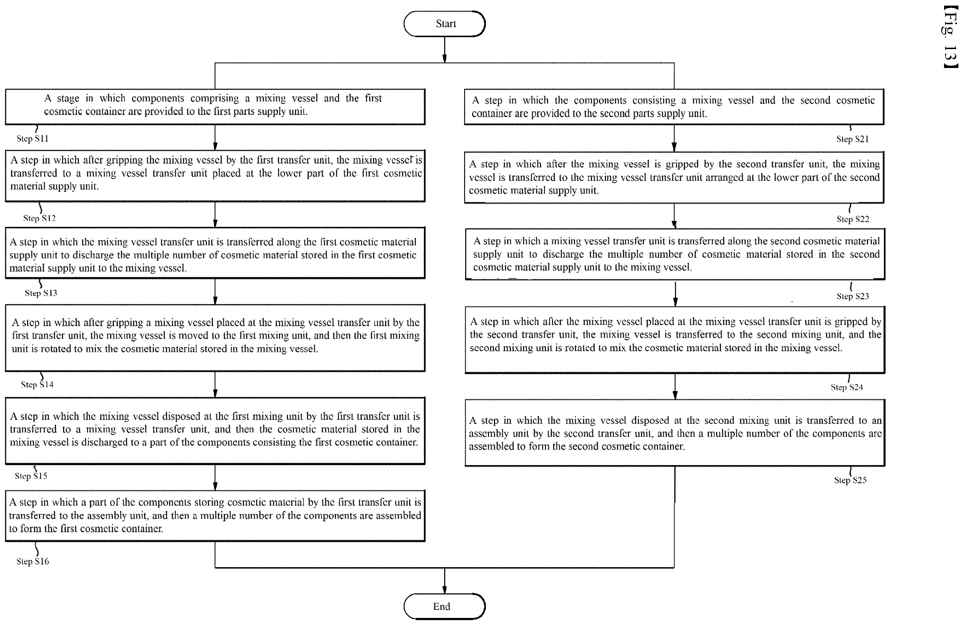

Figure 13 is a flowchart depicting the control method of an apparatus for manufacturing cosmetic in Figure 2.

Hereinafter, the control method of an apparatus for manufacturing cosmetic will be explained with reference to Figure 1 to Figure 13.

The control method of an apparatus for manufacturing cosmetic allows the simultaneous assembly of the first cosmetic container and the second cosmetic container from components. However, the idea of the present invention is not limited thereto and can include the respective manufacturing of the first cosmetic container and the second cosmetic container from components.

First, the process of the first cosmetic container being assembled by an apparatus for manufacturing cosmetic 10 will be explained.

A control method of an apparatus for manufacturing cosmetic can include a step(Step S11) in which a mixing vessel 161 and the component comprising the first cosmetic container are provided to the first parts supply unit 162; a step(Step S12) in which the mixing vessel 161 is gripped by the first transfer unit 810, and then the mixing vessel 161 is transferred to a mixing vessel transfer unit 250 arranged at the lower part of the first cosmetic material supply unit 210; a step(Step S13) in which the mixing vessel transfer unit 250 is transferred along the first cosmetic material supply unit 210 to discharge the multiple number of cosmetic material stored in the first cosmetic material supply unit to the mixing vessel 161; a step(Step S14) in which the mixing vessel 161 placed at the mixing vessel transfer unit 250 is gripped by the first transfer unit 810, the first mixing unit 410 is rotated to mix the cosmetic material stored in the mixing vessel 161; a step(Step S15) in which a mixing vessel 161 placed at the first mixing unit 410 is transferred to a mixing vessel transfer unit 250 by the first transfer unit, and then the cosmetic material stored in the mixing container 161 is discharged to a part of the component consisting the first cosmetic container; and a step(Step S16) in which the first cosmetic container is formed by assembling a multiple number of the components.

In addition, the control method of an apparatus for manufacturing cosmetic can additionally include the step of calculating data on the cosmetic to be manufactured that has a composition ratio by a control unit(not shown in drawing), before the step(Step S11) of providing the component consisting a mixing vessel 161 and the first cosmetic container to the first parts supply unit 162.

Specifically before step S11, a control unit(not shown in drawing) can decide the composition ratio of cosmetic to be manufactured by analyzing the skin condition of a user.

Moreover, a control unit(not shown in drawing) can also decide the composition ratio in consideration of the cosmetic purchase history of a user.

For instance, when there are more purchase histories of a user buying cosmetic of a brighter color than the user's skin condition measured by a skin measurement device 30 or a terminal 20, a control unit(not shown in drawing) can decide a composition ratio to have a brighter color than the composition ratio based on the result of the skin condition analysis.

A control unit, suggesting to a user a decided composition ratio through an interface unit 130, collects the modified data of the composition ratio from the user, and can based on the result thereof adjust the composition ratio and the supply amount of respective cosmetic material.

However, the idea of the present invention is not limited thereto, and a control unit(not shown in drawing) can receive already analyzed composition ratio from a terminal 20 or a skin measurement device 30.

Thereafter, when the composition ratio and total weight of cosmetic to be manufactured are determined, the step(Step S11) of providing a mixing vessel 161 and the components consisting the first cosmetic container to the first parts supply unit 162 can be implemented.

The first parts supply unit 162 can be provided with a base container 1622 which is a component of the first cosmetic container that is a compact-type container, impregnation chamber 1624, cover accommodation part 1625, and mixing vessel 161.

The components described above can directly be placed at the first parts supply unit 162 by a user, or arranged by a separate device.

Next, a step(Step S12) can be implemented in which the first transfer unit 810 grips a mixing vessel 161, and the mixing vessel 161 is transferred to a mixing vessel transfer unit 250 disposed at the bottom of the first cosmetic supply unit 210.

Specifically, Step S12 can include, a step in which a mixing vessel 161 placed at the first parts supply unit 161 is gripped by the first transfer unit 810; and a step in which the first transfer unit 810 gripping a mixing vessel 161, after passing through a transfer unit introduction space 209, seats a mixing vessel on a mixing vessel seating part 252 of a mixing vessel transfer unit 250 placed at the bottom of the first cosmetic material supply unit 210.

Hereinafter, a step(Step S13) can be implemented where a mixing vessel transfer unit 250 can be transferred along the first cosmetic material supply unit 210, to discharge the multiple cosmetic materials stored in the first cosmetic material supply unit 210 to the above depicted mixing vessel 161.

Specifically, Step S13 can include, a step in which a mixing vessel transfer unit 250 is stopped at the bottom of the first cosmetic material supply unit 210 that discharges a single cosmetic material; a step in which a preset amount of cosmetic material is discharged from the first cosmetic material supply unit storing a single cosmetic material to a mixing vessel 161 seated on a mixing vessel transfer unit 250; a step in which a mixing vessel transfer unit 250 is transferred to the bottom of the first cosmetic material supply unit 210 that discharges another cosmetic material; a step in which a preset amount of cosmetic material is discharged from the first cosmetic material supply unit 210 storing another cosmetic material to a mixing vessel 161 seated on a mixing vessel transfer unit 250; and a step in which a mixing vessel transfer unit 250 is transferred consecutively from the bottom of the first cosmetic material supply unit 210 storing a multiple number of cosmetic materials.

Subsequently, a step(Step S14) can be implemented where a mixing vessel 161 placed at a mixing vessel transfer unit 250 is gripped by the first transfer unit 810, and the mixing vessel 161 is then transferred to the first mixing unit 410, for the first mixing unit 410 to be rotated to mix the cosmetic material stored in the mixing vessel 161.

Specifically, Step S14 can include a step in which a mixing unit 161 is transferred to the first mixing unit 410 after gripping a mixing unit 161 placed at a mixing unit transferring unit 250 by the first transfer unit 810; and a step in which cosmetic material stored in a mixing vessel 161 is mixed by rotating the first mixing unit 410 by a preset speed and a preset amount of time.

Next, a step(Step S15) can be included where the first transfer unit 810 transfers a mixing vessel 161 disposed at the first mixing unit 410 to a mixing vessel transfer unit 250, and discharges cosmetic stored in a mixing vessel 161 as part of the components consisting the first cosmetic container.

Specifically, Step S15 can include, a step in which a mixing vessel 161 placed at the first mixing unit 410 by the first transfer unit 810 is transferred to a mixing vessel seating part 252 of a mixing vessel transfer unit 250 placed at the bottom of the first cosmetic material supply unit 210; a step in which a base container 1622 which is a part of the component arranged at the first parts supply unit 162 by the first transfer unit 810 is transferred to a component seating part 256 of a mixing vessel transfer unit 250; a step in which a mixing vessel 161 is arranged inside a vibration gripper.

Hereinafter, after a part of the components in which cosmetic materials are stored is transferred to the assembly unit 50 by the first transfer unit 810, a step(Step S16) that assembles multiple components to form the first cosmetic container can be implemented.

Specifically, Step S16 includes, a step in which a base container 1622 storing the cosmetic material seated on a mixing vessel transfer unit 250 is seated on the first assembly unit 500; a step in which an impregnation member 1624 placed at the first parts supply unit 162 by the first transfer unit 810 is gripped and then the impregnation member 1624 is transferred and inserted into a base container 1622 placed at the first assembly unit 810; a step in which a base container 1622 placed at the first assembly unit 500 by the first transfer unit 810 is transferred to a base container seating part 702 of a base container transfer unit 700 placed at the lower part of a table 52; a step in which a base container seating part 702 of a base container transfer unit 700 is transferred to the bottom of a hygroscopic mechanism 900; a step in which an impregnation member inserted into the inside of a base container 1622 and cosmetic material are mixed by descending the pressing portion 902 of a hygroscopic mechanism 900; a step in which a base container 1622 is transferred to the first assembly unit 500 by gripping a base container 1622 placed at a base container seating part 702 of a base container transfer unit 700; a step in which the first cosmetic container is formed from assembling a cover 1626 and a base container 1622 by transferring a cover 1626 after gripping a cover 1626 placed at the first parts supply unit 162 by the first transfer unit 810; and a step in which the first cosmetic container formed by the first transfer unit 810 is transferred to the first parts supply unit 162.

Next, the process where the second cosmetic container is assembled by an apparatus for manufacturing cosmetic 10 will be explained.

The control method of the apparatus for manufacturing cosmetic can include, a step(Step S21) in which the components comprising a mixing vessel 161 and the second cosmetic container are provided to the second parts supply unit; a step(Step S22) in which the mixing vessel 161 is gripped by the second transfer unit 820, and then the mixing vessel 161 is transferred to a mixing vessel transfer unit 250 disposed at the lower part of the second cosmetic material supply unit 220; a step(Step S23) in which the mixing vessel transfer unit 250 is transferred along the second cosmetic material supply unit 220 to discharge the multiple number of cosmetic material stored in the second cosmetic material supply unit 220 to the mixing vessel 161; a step(Step S24) in which the cosmetic material stored in the mixing vessel is mixed by rotating the second mixing unit 420 after gripping the mixing vessel 161 disposed at the mixing vessel transfer unit 250 by the second transfer unit; and a step(Step S25) in which the second cosmetic container is formed by assembling a multiple number of the components, after transferring to the assembly unit 50 the mixing vessel 161 disposed at the second mixing unit 420 by the second transfer unit 820.

First, the detailed explanation on a step(Step S21) in which the components consisting a mixing vessel 161 and the second cosmetic container are provided to the second parts supply unit is as follows:
A discharge member 1640 and mixing vessel 161, which are the components of the second cosmetic container, which is a pump-type container, can be provided to the second parts supply unit 164.

The above described components can be directly placed at the second parts supply unit 164 by the user, or can be placed by a separate device.

Subsequently, a step(Step S22) where a mixing vessel 161 is gripped by the second transfer unit 820, and then the aforementioned mixing vessel 161 is transferred to a mixing vessel transfer unit 250 disposed at the bottom of the second cosmetic material supply unit 220 can be implemented.

Specifically, Step S22 can include, a step of gripping a mixing vessel 161 placed at the second parts supply unit 164 by the second transfer unit 820; and a step where the first transfer unit 810 gripping a mixing vessel 161 passes through a transfer unit introduction unit 209, and then arranges a mixing vessel 161 at a mixing vessel seating part 252 of a mixing vessel transfer unit 250 disposed at the bottom of the second cosmetic supply unit 220.

Next, a step(Step S23) in which the described mixing vessel transfer unit 250 is moved along with the mentioned second cosmetic supply unit 220, to discharge the multiple cosmetic materials stored in the second cosmetic material supply unit 220 to the mixing vessel 161 can be implemented.

Specifically, Step S23 can include, a step in which a mixing vessel transfer unit 250 is stopped at the bottom of the second cosmetic material supply unit 220 that discharges a single cosmetic material; a step in which a preset amount of cosmetic material is discharged from the second cosmetic material supply unit 220 storing a single cosmetic material to a mixing vessel 161 seated on a mixing vessel transfer unit 250; a step in which a mixing vessel transfer unit 250 is transferred to the bottom of the second cosmetic material supply unit 220 discharging another cosmetic material; a step in which a preset amount of cosmetic material is discharged from the second cosmetic material supply unit 220 storing another cosmetic material to a mixing vessel 161 seated on a mixing vessel transfer unit 250; a step in which a mixing vessel transfer unit 250 is transferred consecutively at the bottom of the second cosmetic material supply unit 220 storing a multiple number of cosmetic materials.

Hereinafter, a step(Step s24) can be implemented in which the above depicted mixing vessel 161 placed at the described mixing vessel transfer unit 250 is gripped by the second transfer unit 820, and then the second mixing unit 420 is rotated to mix the cosmetic material stored in the mixing vessel 161.

Specifically, Step S24 can include a step in which a mixing vessel 161 disposed at a mixing vessel transfer unit 250 by the second transfer unit 820 is gripped to transfer a mixing vessel 161 to the second mixing unit 420; and a step in which the cosmetic material stored in a mixing vessel 161 are mixed by rotating the second mixing unit 420 by a preset speed and preset amount of time.

Hereinafter, a step(Step S25) where a mixing vessel 161 disposed at the second mixing unit 420 by the second transfer unit 820 is transferred to an assembly unit 50, and then multiple components are assembled to form the second cosmetic container can be included.

Specifically, Step S25 can include, a step in which a mixing vessel 161 arranged at the second mixing unit 420 by the second transfer unit 820 is transferred to the second assembly unit 600 of the assembly unit 50; a step in which a discharge member 1640 is transferred to a mixing vessel 161 arranged at the second assembly unit 600 when a discharge member 1640 at the second parts supply unit 164 is gripped by the second transfer unit 820; a step in which the second cosmetic container is formed when assembling a mixing vessel 161 placed at the second assembly unit 600 and a discharge member 1640 by descending and rotating a discharge member 1640 through the second transfer unit 820; and a step in which the second cosmetic container formed completely by the second transfer unit 820 is transferred to the second parts supply unit 164.

The first cosmetic container and second cosmetic container can simultaneously be assembled by the concurrent implementation of the above described Step S11 to Step S16 and Step S21 to Step S25.

Hereinafter is a list of the above described embodiments.

Item 1: In accordance with one aspect of the present invention, an apparatus for manufacturing cosmetic can be provided including, a main body providing a space for manufacturing cosmetic; a parts supply unit providing a mixing vessel that can store cosmetic materials and a components consisting a plurality of cosmetic containers; a cosmetic material supply unit that can store cosmetic materials and then discharge cosmetic materials to the mixing vessel; a transfer unit that can transfer the mixing vessel and the components consisting the cosmetic container; and a control unit that can control the movement of the transfer unit in order to simultaneously assemble a plurality of cosmetic containers from the mixing vessel and the components

Item 2: An apparatus for manufacturing cosmetic of Item 1, wherein a multiple number of mixing vessels are provided to the parts supply unit, and the apparatus for manufacturing cosmetic further includes a mixing unit that can mix the cosmetic material stored in the mixing vessel by rotation, wherein the mixing unit includes: a first mixing unit that mixes a one mixing vessel ; and a second mixing unit that mixes another mixing vessel.

Item 3: An apparatus for manufacturing cosmetic of Item 1 or Item 2, wherein the cosmetic material supply unit includes: a first cosmetic material supply unit that can discharge cosmetic material to a one mixing vessel; and a second cosmetic material supply unit that can discharge cosmetic material to another mixing vessel, wherein the respective first cosmetic material supply unit and the second cosmetic material supply unit include: a plurality of cartridges that can store cosmetic materials; a discharge nozzle that is connected to one side of respective cartridge and discharges cosmetic material to the mixing vessel; a pressure adjustment unit that can discharge the stored cosmetic material to the outside of the discharge nozzle by applying pressure on the inside of the cartridge; and a supply amount adjustment unit that adjusts the discharge amount of the cosmetic material by closing or opening the pathway in which cosmetic material moves, at the discharge nozzle.

Item 4: An apparatus for manufacturing cosmetic according to any one of Item 1 to Item 3, wherein the cosmetic container includes: a first cosmetic container having a first shape; and a second cosmetic container having a second shape, wherein the parts supply unit includes: a first parts supply unit where the components consisting the first cosmetic container are arranged; and a second parts supply unit where the components consisting the second cosmetic container are arranged.

Item 5: An apparatus for manufacturing cosmetic according to any one of Item 1 to Item 4, wherein the first cosmetic container is provided as a compact-type container, wherein the components consisting the compact-type container include: a base container to which the cosmetic material stored in the mixing vessel is transferred and saved; an impregnation member that is inserted in the base container to absorb cosmetic material stored in the base container; and a cover which can cover the impregnation member housed in the base container, wherein the first parts supply unit includes: a base container accommodation part on which the base container can be seated; an impregnation member accommodation part on which the impregnation member can be seated; and a first mixing vessel accommodation part on which the mixing vessel can be seated.

Item 6: An apparatus for manufacturing cosmetic according to any one of Item 1 to Item 5, wherein the second cosmetic container is provided as a pump-type container, wherein the components consisting the pump-type container include: the mixing vessel storing cosmetic material; and a discharge member that covers the mixing vessel and discharges the cosmetic material stored in the mixing vessel, wherein the second parts supply unit include: a storage container seating part on which the mixing vessel can be seated; and a discharge member seating part on which the discharge member can be seated.

Item 7: An apparatus for manufacturing cosmetic according to any one of Item 1 to Item 6, wherein the transfer unit includes: a first transfer unit that can assemble the first cosmetic container from the components; and a second transfer unit that can assemble the second cosmetic container from the mixing vessel and the components.

Item 8: An apparatus for manufacturing cosmetic according to any one of Item 1 to Item 7, further includes an assembly unit providing a space in which a plurality of cosmetic containers are assembled from the mixing vessel and the components, wherein the assembly unit includes: a first assembly unit that provides a space where a first cosmetic container having a first shape is assembled; a second assembly unit that provides a space where a second cosmetic container having a second shape is assembled; a base container transfer unit that can transfer a base container which is a part of component comprising the first cosmetic container; a hygroscopic mechanism that can apply pressure on an impregnation member stored in the base container.

Item 9: An apparatus for manufacturing cosmetic according to any one of Item 1 to Item 8, further includes, a mixing vessel transfer unit that is placed on the lower side of the cosmetic material supply unit and can transfer the mixing vessel.

Item 10: An apparatus for manufacturing cosmetic according to any one of Item 1 to Item 9, further includes a vibrating device that can discharge the cosmetic material stored in the mixing vessel to a part of the components, after gripping the mixing vessel disposed at the mixing vessel transfer unit.

Item 11: An apparatus for manufacturing cosmetic according to any one of Item 1 to Item 10, wherein the cosmetic material supply unit includes a plurality of cartridges that can store the cosmetic materials, and between the plurality of cartridges, a space for introducing a transfer unit that allows the entrance of the transfer unit gripping the mixing vessel is formed.

Item 12: A control method of an apparatus for manufacturing cosmetic including, (S11) a step in which the components comprising a mixing vessel and the first cosmetic container are provided to the first parts supply unit; (S12) a step in which after the mixing vessel is gripped by the first transfer unit, the mixing vessel is transferred to a mixing vessel transfer unit placed at the bottom of the first cosmetic material supply unit; (S13) a step in which the mixing vessel transfer unit is transferred along the first cosmetic material supply unit, to discharge the multiple number of cosmetic materials stored in the first cosmetic material supply unit to the mixing vessel; (S14) a step in which after the mixing vessel arranged at the mixing vessel transfer unit is gripped by the first transfer unit, the mixing vessel is transferred to the first mixing unit, and then the first mixing unit is rotated to mix the cosmetic material stored in the mixing vessel; (S15) a step in which after the mixing vessel arranged at the first mixing unit by the first transfer unit is transferred to a mixing vessel transfer unit, the cosmetic material stored in the mixing vessel is discharged as a part of the components consisting the first cosmetic container.

Item 13: A control method of an apparatus for manufacturing cosmetic of Item 12 including, (S21) a step in which the mixing vessel and the components consisting the second cosmetic container are provided to the second parts supply unit; (S22) a step in which after the mixing unit is gripped by the second transfer unit 820, the mixing unit 161 is transferred to the mixing unit transfer unit placed at the bottom of the second cosmetic material supply unit; (S23) a step in which the mixing vessel transfer unit is transferred along the second cosmetic material supply unit to discharge the multiple number of cosmetic material stored in the second cosmetic material supply unit to the mixing vessel; (S24) a step in which after the mixing vessel placed at the mixing vessel transfer unit is gripped by the second transfer unit, the mixing vessel is then transferred to the second mixing unit, to rotate the second mixing unit for mixing the cosmetic material stored in the mixing vessel 161; (S25) a step in which after the mixing vessel disposed at the second mixing unit by the second transfer unit is transferred to an assembly unit, the second cosmetic container is formed by assembling a multiple number of the components.

Item 14: A control method of an apparatus for manufacturing cosmetic of Item 14, wherein the S 1 1 to S 16, and the S21 to 25 are simultaneously implemented.

Item 15: In accordance with one aspect of the present invention, there is provided a vibrating device including: a vibrating gripper capable of gripping a mixing vessel containing cosmeic; a rotary unit that can rotate the vibrating gripper to tilt a mixing vessel gripped by the vibrating gripper; and vibration generation unit that can apply vibration to the vibrating gripper to discharge the cosmetic material stored in the mixing vessel to the outside of the vessel.

Item 16: A vibrating device of Item 15, wherein the vibrating gripper includes, a first vibrating gripper that can come into contact with a part of the mixing vessel; a second vibrating gripper that can come into contact with another part of the mixing vessel, and the first vibrating gripper and second vibrating gripper can move close to or far from each other.

Item 17: A vibrating device of Item 15 or Item 16, wherein the rotary unit includes a rotary member connected to the vibration generation unit, and provided to be rotatable by 360 degrees.

Item 18: A vibrating device according to any one of Item 15 to Item 17, wherein the vibration generation unit includes, a main body of the vibration generation unit connected to the rotary unit; and a multiple number of vibrating members that connects a main body of a vibration generation unit and the above vibrating gripper and can respectively move in different directions.

Item 19: A vibrating device according to any one of Item 15 to Item 18, wherein the vibration member can vibrates in the frequency of 40Hz or 400Hz.

Item 20: In accordance with one aspect of the present invention, there is provided an apparatus for manufacturing cosmetic including, a mixing vessel transfer unit providing a mixing vessel seating part that can be seated with a mixing vessel storing cosmetic material and a component seating part that can be seated with a base container provided as an empty container; and a vibrating device according to any one of Item 15 to Item 19, wherein the vibrating device can transfer the cosmetic material stored in the mixing vessel seated on the mixing vessel transfer unit to the base container by gripping and tilting a mixing vessel seated on the mixing vessel transfer unit.

Item 21: An apparatus for manufacturing cosmetic according to any one of Item 1 to Item 11 and Item 20, further includes an assembly unit providing a space in which the base container can be assembled into the first cosmetic container which is a compact-type container.

Item 22: An apparatus for manufacturing cosmetic according to any one of Item 1 to Item 11, Item 20 and Item 21, wherein the assembly unit including: a first assembly unit that provides a space in which the first cosmetic container is assembled; a base container transfer unit that can transfer the base container into which an impregnation member is inserted; and a hygroscopic device that can apply pressure on an impregnation chamber stored in a base container.

Item 23: An apparatus for manufacturing cosmetic according to any one of Item 1 to Item 11 and Item 20 to Item 22, further inclues a parts supply unit providing a mixing vessel and components consisting the first cosmetic container; a cosmetic supply unit that can , after storing a multiple number of cosmetic materials, discharge the cosmetic materials by the mixing vessel; a transfer unit that can transfer the components consisting the mixing vessel and the first cosmetic container; and a control unit controlling the movement of the transfer unit to assemble the first cosmetic container from the mixing vessel and components.

Although a system for manufacturing cosmetic and an apparatus for manufacturing cosmetic in line with the embodiments of the present invention were explained hereabove, as they are merely examples, the present invention is not limited thereto, and should be construed as having the broadest scope in line with the basic idea described in the present specification. A person skilled in the art may combine or replace the embodiments described to embody any pattern(s) having an unspecified form, but this also falls within the scope of the present invention. Furthermore, while a person skilled in the art may easily change or modify the embodiments described on the basis of the present specification, it is evident that such change or modification falls under the scope of the rights of the present invention.

## Claims

1. An apparatus for manufacturing cosmetic comprising:
a main body 100 providing a space 102 for manufacturing cosmetic;
a parts supply unit 160 providing a mixing vessel 161 that can store cosmetic materials and a components consisting a plurality of cosmetic containers;
a cosmetic material supply unit 200 that can store cosmetic materials and then discharge cosmetic materials to the mixing vessel 161;
a transfer unit 810 that can transfer the mixing vessel 161 and the components consisting the cosmetic container; and
a control unit that can control the movement of the transfer unit 810 in order to simultaneously assemble a plurality of cosmetic containers from the mixing vessel 161 and the components.

2. An apparatus for manufacturing cosmetic of claim 1, wherein a multiple number of mixing vessels 161 are provided to the parts supply unit 160,
and the apparatus for manufacturing cosmetic further includes a mixing unit 400 that can mix the cosmetic material stored in the mixing vessel 161 by rotation,
wherein the mixing unit 400 includes:
a first mixing unit 410 that mixes a one mixing vessel 161 ; and
a second mixing unit 420 that mixes another mixing vessel 161.

3. An apparatus for manufacturing cosmetic of claim 1, wherein the cosmetic material supply unit 200 includes:
a first cosmetic material supply unit 210 that can discharge cosmetic material to a one mixing vessel 161; and
a second cosmetic material supply unit 220 that can discharge cosmetic material to another mixing vessel 161,
wherein the respective first cosmetic material supply unit 210 and the second cosmetic material supply unit 220 include:
a plurality of cartridges 202 that can store cosmetic materials;
a discharge nozzle 204 that is connected to one side of respective cartridge 202 and
discharges cosmetic material to the mixing vessel 161;
a pressure adjustment unit 206 that can discharge the stored cosmetic material to the outside of the discharge nozzle 204 by applying pressure on the inside of the cartridge 202; and
a supply amount adjustment unit 208 that adjusts the discharge amount of the cosmetic material by closing or opening the pathway in which cosmetic material moves, at the discharge nozzle 204.

4. An apparatus for manufacturing cosmetic of claim 1, wherein the cosmetic container includes:
a first cosmetic container having a first shape; and
a second cosmetic container having a second shape,
wherein the parts supply unit 160 includes:
a first parts supply unit 162 where the components consisting the first cosmetic container are arranged; and
a second parts supply unit 164 where the components consisting the second cosmetic container are arranged.

5. An apparatus for manufacturing cosmetic of claim 4, wherein the first cosmetic container is provided as a compact-type container,
wherein the components consisting the compact-type container include:
a base container 1622 to which the cosmetic material stored in the mixing vessel 161 is transferred and saved;
an impregnation member 1624 that is inserted in the base container 1622 to absorb cosmetic material stored in the base container 1622; and
a cover 1626 which can cover the impregnation member 1624 housed in the base container 1622,
wherein the first parts supply unit 162 includes:
a base container accommodation part 1621 on which the base container 1622 can be seated;
an impregnation member accommodation part 124 on which the impregnation member 1624 can be seated; and
a first mixing vessel accommodation part 1627 on which the mixing vessel 161 can be seated.

6. An apparatus for manufacturing cosmetic of claim 4,
wherein the second cosmetic container is provided as a pump-type container,
wherein the components consisting the pump-type container include:
the mixing vessel 161 storing cosmetic material; and
a discharge member 1640 that covers the mixing vessel 161 and discharges the cosmetic material stored in the mixing vessel 161,
wherein the second parts supply unit 164 include:
a storage container seating part 1643 on which the mixing vessel 161 can be seated; and
a discharge member seating part 1645 on which the discharge member 1640 can be seated.

7. An apparatus for manufacturing cosmetic of claim 4,
wherein the transfer unit 800 includes:
a first transfer unit 810 that can assemble the first cosmetic container from the components; and
a second transfer unit 820 that can assemble the second cosmetic container from the mixing vessel 161 and the components.

8. An apparatus for manufacturing cosmetic of claim 1 further includes an assembly unit 50 providing a space in which a plurality of cosmetic containers are assembled from the mixing vessel 161 and the components,
wherein the assembly unit 50 includes:
a first assembly unit 500 that provides a space where a first cosmetic container having a first shape is assembled;
a second assembly unit 600 that provides a space where a second cosmetic container having a second shape is assembled;
a base container transfer unit 700 that can transfer a base container 1622 which is a part of component comprising the first cosmetic container;
a hygroscopic mechanism 900 that can apply pressure on an impregnation member 1624 stored in the base container 1622.

9. An apparatus for manufacturing cosmetic of claim 1 further includes, a mixing vessel transfer unit 250 that is placed on the lower side of the cosmetic material supply unit and can transfer the mixing vessel.

10. An apparatus for manufacturing cosmetic of claim 1 further includes a vibrating device 300 that can discharge the cosmetic material stored in the mixing vessel 161 to a part of the components, after gripping the mixing vessel 161 disposed at the mixing vessel transfer unit 250.

11. An apparatus for manufacturing cosmetic of claim 1,
wherein the cosmetic material supply unit includes a plurality of cartridges 202 that can store the cosmetic materials, and between the plurality of cartridges 202, a space for introducing a transfer unit 209 that allows the entrance of the transfer unit 800 gripping the mixing vessel 161 is formed.

12. A control method of an apparatus for manufacturing cosmetic including,
(S11) a step in which the components comprising a mixing vessel 161 and the first cosmetic container are provided to the first parts supply unit 162;
(S12) a step in which after the mixing vessel 161 is gripped by the first transfer unit 810, the mixing vessel 161 is transferred to a mixing vessel transfer unit placed at the bottom of the first cosmetic material supply unit 210;
(S13) a step in which the mixing vessel transfer unit 250 is transferred along the first cosmetic material supply unit 210, to discharge the multiple number of cosmetic materials stored in the first cosmetic material supply unit 210 to the mixing vessel 161;
(S14) a step in which after the mixing vessel 161 arranged at the mixing vessel transfer unit 250 is gripped by the first transfer unit 810, the mixing vessel 161 is transferred to the first mixing unit 410, and then the first mixing unit 410 is rotated to mix the cosmetic material stored in the mixing vessel 161;
(S15) a step in which after the mixing vessel 161 arranged at the first mixing unit by the first transfer unit is transferred to a mixing vessel transfer unit 250, the cosmetic material stored in the mixing vessel 161 is discharged as a part of the components consisting the first cosmetic container.

13. A control method of an apparatus for manufacturing cosmetic of claim 12 including (S21) a step in which the mixing vessel 161 and the components consisting the second cosmetic container are provided to the second parts supply unit 164;
(S22) a step in which after the mixing unit 161 is gripped by the second transfer unit 820, the mixing unit 161 is transferred to the mixing unit transfer unit placed at the bottom of the second cosmetic material supply unit 220;
(S23) a step in which the mixing vessel transfer unit 250 is transferred along the second cosmetic material supply unit 220 to discharge the multiple number of cosmetic material stored in the second cosmetic material supply unit to the mixing vessel 161;
(S24) a step in which after the mixing vessel 161 placed at the mixing vessel transfer unit 250 is gripped by the second transfer unit 820, the mixing vessel 161 is then transferred to the second mixing unit 420, to rotate the second mixing unit for mixing the cosmetic material stored in the mixing vessel 161;
(S25) a step in which after the mixing vessel 161 disposed at the second mixing unit 420 by the second transfer unit 820 is transferred to an assembly unit 50, the second cosmetic container is formed by assembling a multiple number of the components.

14. A control method of an apparatus for manufacturing cosmetic of claim 14, wherein the S11 to S 16, and the S21 to 25 are simultaneously implemented.
